# EUROPEAN PATENT SPECIFICATION

(11) **EP 1 945 660 B1**
(45) Date of publication and mention of the grant of the patent: **07.05.2014**
(21) Application number: 06790746.9
(22) Date of filing: 26.09.2006
(51) Int. Cl.: C07K 14/245, C12Q 1/18, C40B 30/04, C40B 40/10, G01N 33/566, G01N 33/58

(54) **LIBRARY FROM TOXIN MUTANTS, AND METHODS OF USING SAME**
BIBLIOTHEK AUS TOXINMUTANTEN UND VERFAHREN ZU DEREN ANWENDUNG
BANQUES FORMÉES À PARTIR DE TOXINES MUTANTES, ET PROCÉDÉS D'UTILISATION DE CELLES-CI

(30) Priority: 26.09.2005 US 720924 P
(43) Date of publication of application: 23.07.2008
(60) Divisional application: 11168475.9
(73) Proprietor: Molecular Templates, Inc., Georgetown, TX 78626 (US)
(72) Inventor: GARIEPY, Jean, Toronto, Ontario M5P 2L5 (CA); REVERS, Leigh, Toronto, Ontario M4Y 3C4 (CA)
(74) Representative: Griffin, Philippa Jane
(86) International application number: PCT/CA2006/001581
(87) International publication number: WO 2007/033497

(56) References cited:
- WO-A1-99/40185
- WO-A1-2005/092917
- US-A- 5 552 144
- DERESIEWICZ R L ET AL: "MUTATIONS AFFECTING THE ACTIVITY OF THE SHIGA-LIKE TOXIN I A-CHAIN" BIOCHEMISTRY, vol. 31, no. 12, 1992, pages 3272-3280, XP002509633 ISSN: 0006-2960
- PERAMPALAM, S: "Cell-targeted ribosome-inactivating proteins derived from protein combinatorial libraries"[Online] pages 1-158, XP002509634 Retrieved from the Internet: URL:https://tspace.library.utoronto.ca/bit stream/1807/11244/1/perampalam_subodini_20 0804_PhD_thesis.pdf> [retrieved on 2009-01-09]
- BRAY M.R. ET AL.: 'Probing the surface of eukaryotic cells using combinatorial toxin libraries' CURRENT BIOLOGY vol. 11, no. 9, 01 May 2001, pages 697 - 701, XP002303223
- PERAMPALAM S ET AL: "Designing combinatorial protein libraries based on a protein toxin template", MOLECULAR & CELLULAR PROTEOMICS, AMERICAN SOCIETY FOR BIOCHEMISTRY AND MOLECULAR BIOLOGY, INC, US, vol. 2, no. 9, 11 January 2003 (2003-01-11), page 868, XP009110452, ISSN: 1535-9476
- LACY D B ET AL: "Unraveling the structures and modes of action of bacterial toxins.", CURRENT OPINION IN STRUCTURAL BIOLOGY DEC 1998, vol. 8, no. 6, December 1998 (1998-12), pages 778-784, ISSN: 0959-440X
- STROCKBINE N A ET AL: "Cloning and sequencing of the genes for Shiga toxin from Shigella dysenteriae type 1.", JOURNAL OF BACTERIOLOGY MAR 1988, vol. 170, no. 3, March 1988 (1988-03), pages 1116-1122, ISSN: 0021-9193

## Description

### Background of the Invention

This application relates to libraries of toxin mutants, and to methods of using same in the development of therapeutics targeted against specific cell types.

Plant and bacterial toxins have a structural organization with two or more polypeptide domains or subunits responsible for distinct functions, referred to as A and B. The toxins may be referred to as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin A (Olsnes, S. and Sandvik, K. (1988) in Immunotoxins pp. 39-73, Kluwer Academic, Boston; Sandvik, K., Dubinina, E., Garred, O., et al. (1992) Biochem. Soc. Trans. 20:724) as well as plant toxins such as ricin and abrin. In some cases the toxin are heteromeric, in that the B chains are actually separate entities that connect to the toxic A chain via a non-covalent bonding. In other cases, the toxin is monomeric, since the B chain is part of the same protein when the toxin is produced in nature. In many cases, the A chain has been characterized as having two domains, an A1 domain and an A2 domain.

Based on their ability to block protein synthesis, proteins such as Shiga and Shiga-like toxins as well as ricin, abrin, gelonin, crotin, pokeweed antiviral protein, saporin, momordin, modeccin, sarcin, diphtheria toxin and exotoxin A have been referred to as ribosome-inactivating proteins (RIP). The potency of RIPs is exceedingly high; one molecule of diphtheria toxin A chain (Yamaizumi, et al. (1978) Cell 15:245-250) or ricin A chain (Eiklid, et al. (1980) Exp. Cell Res. 126:321-326) having been shown to be sufficient to kill a eukaryotic cell.

International Patent Publication No. WO99/4018 describes libraries of mutant toxins in which mutations are introduced into the binding domain to alter the type of cells to which the toxic species are delivered. The new proteins are derived by mutating a binding subunit of the wild type heteromeric protein cytotoxic protein to create a library of microorganism clones producing mutant proteins, which are then screened for the ability to specifically bind to and kill a target cell type.

US Patent No. 5,552,144 discloses a Shigella-like toxin II variant to which a mutation is introduced into the A chain at position 167 to change the amino acid at this position to one with a different charge. This resulted in a toxin with less of the enzymatic activity associated with toxicity.

US Patent No. 6,593,132 describes recombinant toxic proteins which are specifically toxic to diseased cells but do not depend for their specificity of action on a specific cell binding component. The recombinant proteins of the '132 patent have an A chain of a ricin-like toxin linked to a B chain by a synthetic linker sequence which may be cleaved specifically by a protease localized in cells or tissues affected by a specific disease to liberate the toxic A chain thereby selectively inhibiting or destroying the diseased cells or tissues.

US Patent No. 6,649,742 discloses Type I ribosome-inactivating proteins (RIPs) and analogs of the RIPs having a cysteine available for disulfide bonding to targeting molecules. The RIPs and RIP analogs are used as components of cytotoxic therapeutic agents to selectively eliminate any cell type to which the RIP component is targeted by the specific binding capacity of the second component of the agent.

PCT application PCT/CA2004/000433 by the present inventors discloses combinatorial protein libraries comprising a plurality of protein species, in which each protein species comprises an A chain of a heteromeric toxic protein, into which an insert has been introduced.

Bray et al., Current Biology, Vol. 11(9), 1 May 2001, pages 697-701, describes probing the surface of eukaryotic cells using combinatorial toxin libraries.

Perampalam et al., Molecular and Cellular Proteomics, Vol. 2(9), 11 January 2003, page 868, describes designing combinatorial protein libraries based on a protein toxin template.

Lacy et al., Current Opinion in Structural Biology, Vol. 8(6), Dec 1998, pages 778-784, describes unravelling the structures and modes of action of bacterial toxins.

An aspect of the present invention is a combinatorial protein library as defined in the claims, comprising a plurality of protein species, each protein species comprising an A chain of a Shiga-like toxin 1 protein into which an insert has been introduced, wherein, (a) the insert is a polypeptide of varying amino acid sequence having a length of 3-200 amino acid residues; and (b) the insert is introduced into the protease-sensitive loop of the A chain sequence. In a further embodiment, the library may comprise up to or over 100 protein species. The insert is introduced into the Shiga-like toxin IA chain between amino acids 242 and 261, such as between amino acids 245 and 246.

In a further embodiment of the present invention, the insert has a length of 7 amino acids.

Another aspect of the present invention is a mutant protein as defined in the claims, comprising an A chain of a Shiga-like toxin 1 protein into which an insert has been introduced, wherein the insert is a polypeptide of varying amino acid sequence having a length of 3-200 amino acid residues; and the insert is introduced into the protease-sensitive loop of the A chain sequence. The insert is introduced between amino acids 242 and 261, such as between amino acids 245 and 246.

A further aspect described herein is a method for identifying a ligand that binds to a specific target/receptor, comprising the steps of exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

A further aspect of the present invention is a method, as defined in the claims, for isolating a toxin specific for a known target/receptor comprising the steps of: exposing the target/receptor to a combinatorial protein library as defined in the claims; and isolating at least one protein species from the combinatorial protein library captured by binding to the target/receptor.

A further aspect of the present invention is a combinatorial protein library as defined in the claims, comprising a plurality of protein species, each protein species comprising a modified A chain of a Shiga-like toxin 1 protein into which an insert has been introduced, wherein, the modified A chain comprises a wild-type A chain containing a mutation in at least one cysteine (as defined in the claims); the insert is a polypeptide of varying amino acid sequence having a length of 2-300 amino acid residues; and the insert is introduced into the region which would be a protease-sensitive loop in the wild-type A chain sequence.

A further aspect of the present invention is a mutant protein as defined in the claims, comprising a modified A chain of a Shiga-like toxin 1 protein into which an insert has been introduced, wherein, the modified A chain comprises a wild-type A chain containing a mutation in at least one cysteine (as defined in the claims); the insert is a polypeptide of varying amino acid sequence having a length of 2-300 amino acid residues; and the insert is introduced into a region which would be a protease-sensitive loop in the wild type A chain sequence. In a further embodiment, the modified A chain further comprises a label bound to a cysteine.

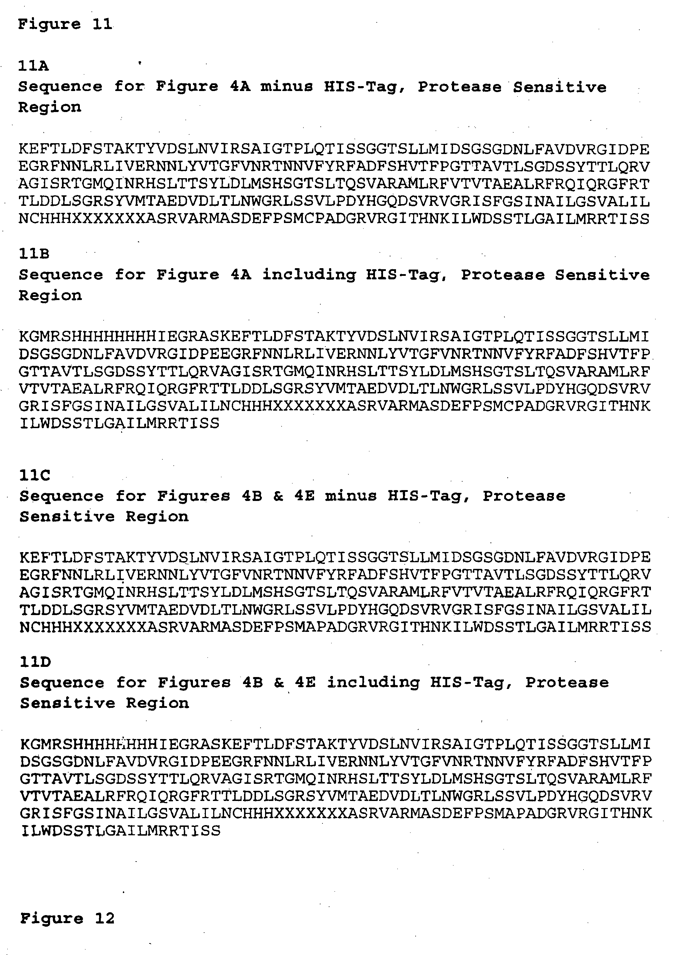

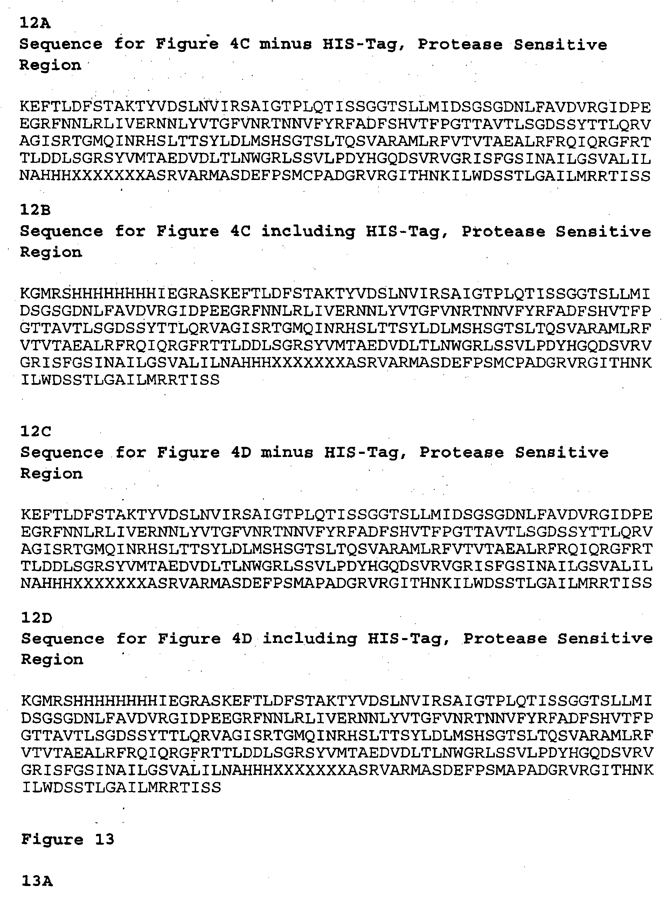

The specification provides a polypeptide sequence selected from the group consisting of the polypeptide sequence listed in figures 11C, 11D, 12A, 12B, 12C, or 12D.

The specification provides method for identifying a ligand that bind to a specific target/receptor, comprising the steps of: exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

An other embodiment of the present invention is a method as defined in the claims, for isolating a toxin specific for a known target/receptor comprising the steps of exposing the target/receptor to a combinatorial protein library as defined in the claims; and isolating at least one protein species from the combinatorial protein library captured by binding to the target/receptor.

The specification provides a combinatorial protein library comprising a plurality of protein species, each protein species comprising an A1 chain of a toxic protein into which an insert has been introduced, wherein, the insert is a polypeptide of varying amino acid sequence having a length of at least 2 amino acid residues; and the insert is introduced into the portion of the A1 chain that would be a protease-sensitive loop if the A1 chain was a full-length A chain.

The specification provides a mutant protein comprising an A1 chain of a toxic protein into which an insert has been introduced, wherein, the insert is a polypeptide of varying amino acid sequence having a length of at least 2 amino acid residues; and the insert is introduced into the portion of the A1 chain that would be a protease-sensitive loop if the A1 chain was a full length A chain.

The specification provides a method for identifying a ligand that bind to a specific target/receptor, comprising the steps of: exposing cells known to possess the target/receptor to members of a combinatorial protein library as described herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

The specification provides a combinatorial protein library comprising a plurality of protein species, each protein species comprising a modified A1 chain of a toxic protein into which an insert has been introduced, wherein, the modified A1 chain comprises a wild-type A1 chain containing a mutation in one cysteine (as defined in the claims); the insert is a polypeptide of varying amino acid sequence having a length of at least 2 amino acid residues; and the insert is introduced into the portion of the A1 chain that would be a protease-sensitive loop if the A1 chain was a full length A chain.

The specification provides a mutant protein comprising a modified A1 chain of a toxic protein into which an insert has been introduced, wherein, the modified A1 chain comprises a wild-type A1 chain containing a mutation in one cysteine (as defined in the claims); the insert is a polypeptide of varying amino acid sequence having a length of at least 2 amino acid residues; and the insert is introduced into the portion of the A1 chain that would be a protease-sensitive loop if the A1 chain was a full-length A chain.

The specification provides a method for identifying a ligand that bind to a specific target/receptor, comprising the steps of: exposing cells known to possess the target/receptor to members of a combinatorial protein library as disclosed herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

The specification describes a combinatorial protein library that has the insert introduced at the N-terminus of the A chain sequence instead of in the protease sensitive loop region.

The specification describes a method for identifying a ligand that bind to a specific target/receptor, comprising the steps of: exposing cells known to possess the target/receptor to members of a combinatorial protein library as disclosed herein; selecting members of the protein library which are observed to be toxic to the cells; evaluating the selected members of the protein library to determine the sequence of the inserted region, whereby a peptide of the sequence of the inserted region is identified as a possible ligand for a target/receptor on the cell; and further testing peptides of the sequence of the inserted region to confirm that they are a ligand for the specific target/receptor.

### Brief Description of the Drawings

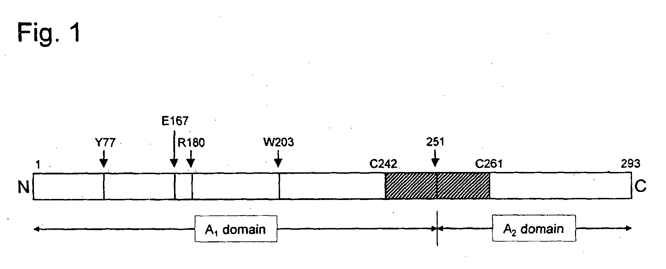

Fig. 1 is a schematic diagram depicting the A1 and A2 domains of wild-type SLT-1. The A chain is composed of 293 amino acids. The chain is cleaved by furin to produce an A1 catalytic fragment and an A2 C-terminal tail non-covalently associated with the B pentamer. A protease-sensitive loop (stripped area) is defined by the only two cysteine residues in the A chain (Cys 242 and 261). Tyr77, Glu167, Arg170 and Trp203 represent residues important for the catalytic activity of the A1 domain (arrows).

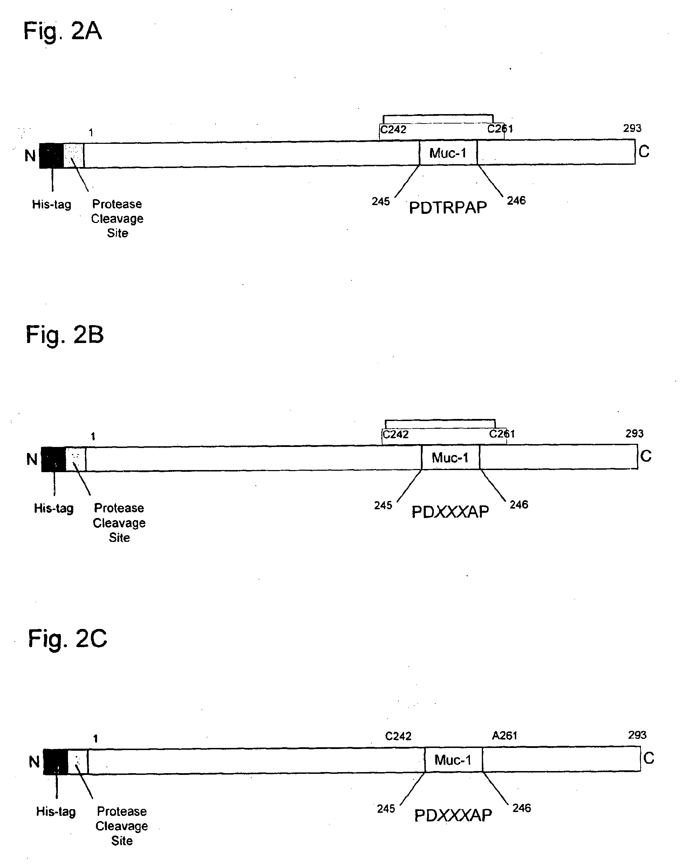

Fig. 2A is a schematic representation of the SLT-1 A chain (1-293) with the breast cancer-associated MUCl epitope PDTRPAP (control sequence recognized by the mAb One M27) inserted between residues 245 and 246 and a 6-Histidine tag followed by a protease cleavage site (such as a furin or thrombin cleavage site) at its N-terminus.
Fig. 2 B is a depiction of a SLT-1 A chain-tripeptide library construction where the three positions of the MUCl epitope recognized by the mAb One M27 are randomized (XXX region). The tripeptide library was inserted in a naturally occurring loop region of the A chain created by the presence of a disulfide bridge between Cys 242 and Cys 261.
Fig. 2 C is a depiction of the SLT-1 A chain tripeptide library construction of Figure 2 B, wherein cysteine 261 has been mutated to an alanine. Note that in this case (as in many of the figures below) the disulfide bridge present in Fig. 2B can not be formed due to the absence of one of the cysteines.

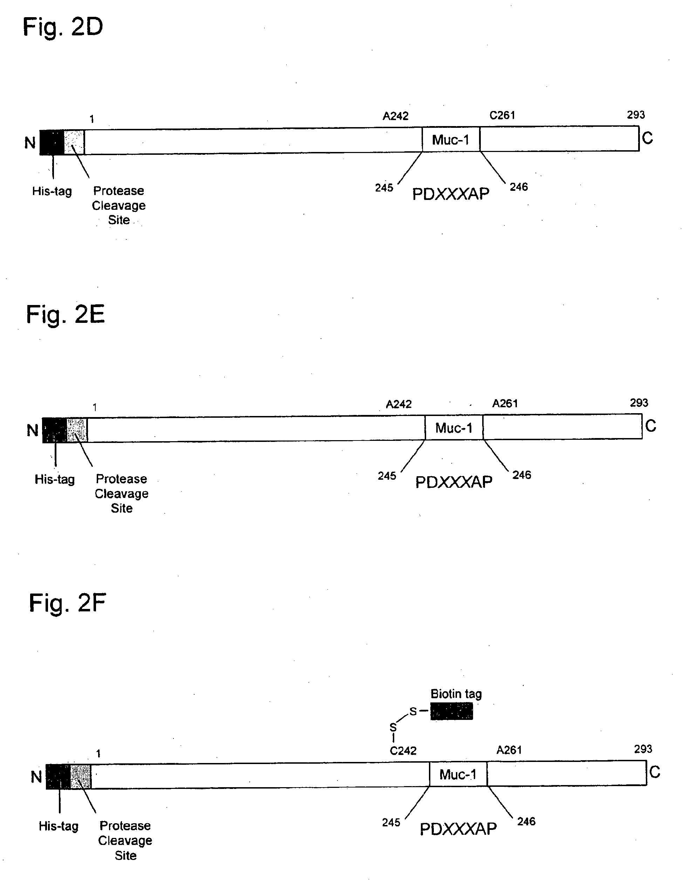

Figure 2D is a depiction of the SLT-1 A chain tripeptide library construction of Figure 2B, wherein cysteine 242 has been mutated to an alanine.
Figure 2E is a depiction of the SLT-1 A chain tripeptide library construction of Figure 2B, wherein cysteine 242 and cysteine 261 have both been mutated to alanine residues.
Figure 2F is a depiction of the SLT-1 A chain tripeptide library construction of Figure 2C, wherein the C242 residue has been biotinylated.

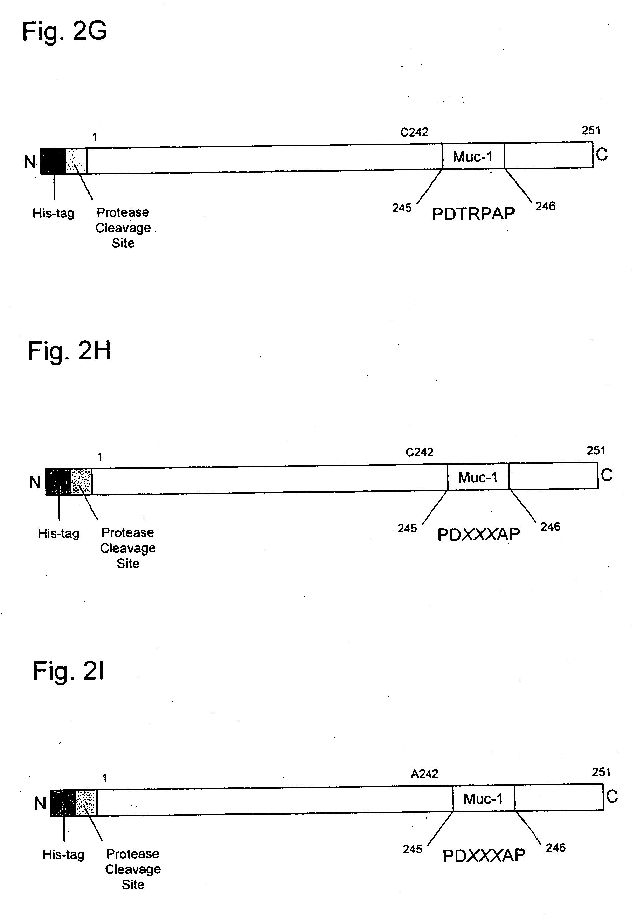

Figure 2G is a schematic representation of the SLT-1 A1 chain (1-251) with the breast cancer-associated MUC1 epitope PDTRPAP (control sequence recognized by the mAb One M27) inserted between residues 245 and 246 and a Histidine tag followed by a protease cleavage site (such as a furin or thrombin cleavage site) at its N-terminus.
Fig. 2 H is a depiction of our SLT-1 A1 chain-tripeptide library construction where the three key positions of the MUCl epitope recognized by the mAb One M27 were randomized (XXX region).
Fig. 2 I is a depiction of the SLT-1 A1 chain tripeptide library construction of Figure 2 H, wherein cysteine 242 has been mutated to an alanine.
Figure 2J is a depiction of the SLT-1 A1 chain tripeptide library construction of Figure 2H, wherein cysteine 242 has been biotinylated.
Fig. 3 shows a representative ELISA data set from screening 96 distinct single A chain variants from our SLT-1 A chain-tripeptide library with mAb Onc M27. Toxin variant # 41 (Tables 1 and 2) gave a strong ELISA signal and had the expected epitope.
Fig. 4A shows a schematic diagram of a 7-amino acid random segment inserted between residues 245 and 246 of the A chain.
Fig. 4B shows a schematic diagram of a 7-amino acid random segment inserted between residues 245 and 246 of the A chain, wherein the cysteine at position 261 has been mutated to an alanine.
Fig. 4C shows a schematic diagram of a 7-amino acid random segment inserted between residues 245 and 246 of the A chain, wherein the cysteine at position 242 has been mutated to an alanine.
Fig. 4D shows a schematic diagram of a 7-amino acid random segments inserted between residues 245 and 246 of the A chain, wherein the cysteine at positions 242 and 261 have been mutated to alanines.
Fig. 4E shows a schematic diagram of a 7-amino acid random segment inserted between residues 245 and 246 of the A chain, wherein the cysteine at position 261 has been mutated to an alanine and the cysteine at position 242 has been biotinylated.
Fig. 4F shows a schematic diagram of a 7-amino acid random segment inserted between residues 245 and 246 of the A1 chain.
Fig. 4G shows a schematic diagram of a 7-amino acid random segment inserted between residues 245 and 246 of the A1 chain, wherein the cysteine residue at position 242 has been biotinylated.
Fig. 4H shows a schematic diagram of a 7-amino acid random segment inserted between residues 245 and 246 of the A1 chain, wherein the cysteine residue at position 242 has been mutated to an analine.
Fig. 5 shows the results of tests on seven toxin variants that were identified as repeatable killers of the human melanoma cell line 518A2. The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).

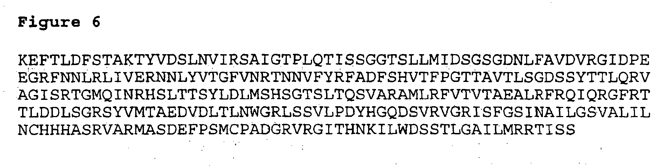

Fig. 6 shows the amino acid sequence corresponding to the wild-type SLT-1 protein of Figure 1. Amino acid sequences are shown from N-terminus to C-terminus.

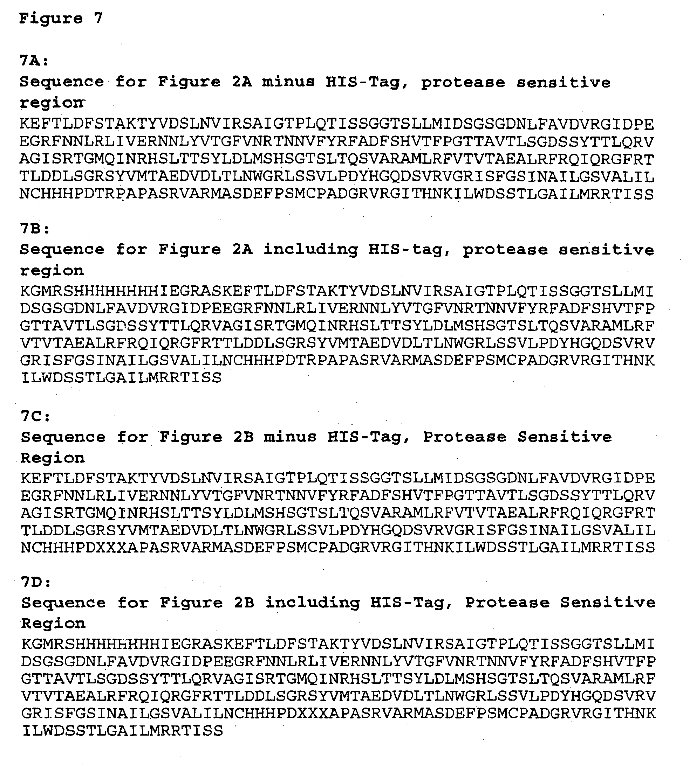

Figure 7A shows the amino acid sequence corresponding to the SLT-A chain of Figure 2A, less the N-terminus tags (the His-tag and the protease cleavage site).
Figure 7B shows the amino acid sequence corresponding to the SLT-A chain of Figure 2A, with an example of one possible N-terminus tag, comprising a 8-His-tag and a protease cleavage site.
Figure 7C shows the amino acid sequence corresponding to the SLT-A chain-tripeptide library construction of Figure 2B, less the N-terminus tags (the His-tag and the protease cleavage site).
Figure 7D shows the amino acid sequence corresponding to the SLT-A chain-tripeptide library construction of Figure 2B, with an example of one possible N-terminus tag, comprising a 8-His-tag and a protease cleavage site.

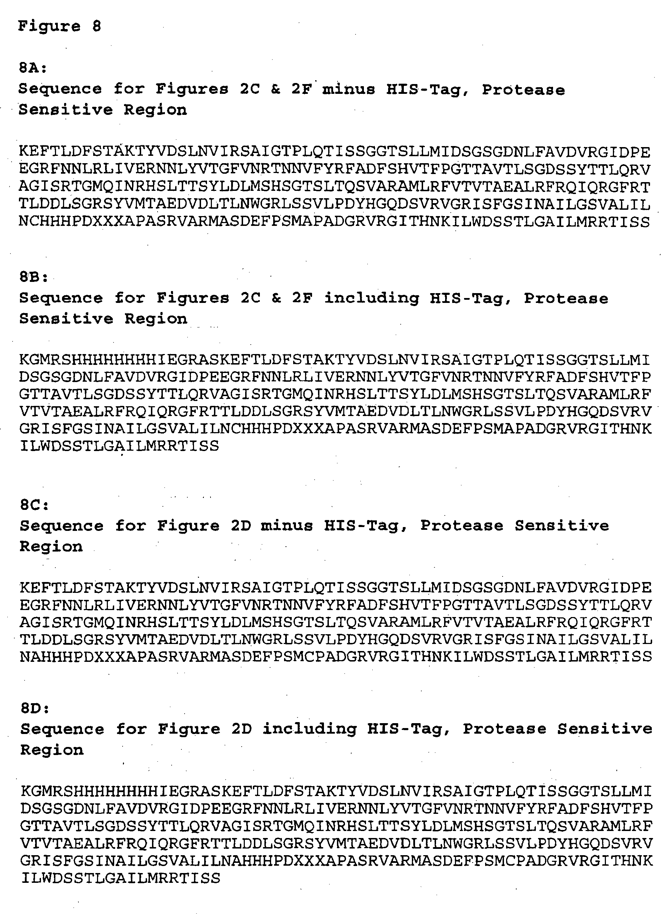

Figure 8A shows the amino acid sequence corresponding to the SLT-1 A chain tripeptide library of Figure 2C and 2F, less the N-terminus tags (the His-tag and the protease cleavage site).
Figure 8B shows the amino acid sequence corresponding to the SLT-1 A chain tripeptide library of Figure 2C and 2F, with an example of one possible N-terminus tag, comprising a 8-His-tag and a protease cleavage site.
Figure 8C shows the amino acid sequence corresponding to the SLT-1 A chain tripeptide library of Figure 2D, less the N-terminus tags (the His-tag and the protease cleavage site).
Figure 8D shows the amino acid sequence corresponding to the SLT-1 A chain tripeptide library of Figure 2D, with an example of one possible N-terminus tag, comprising a 8-His-tag and a protease cleavage site.

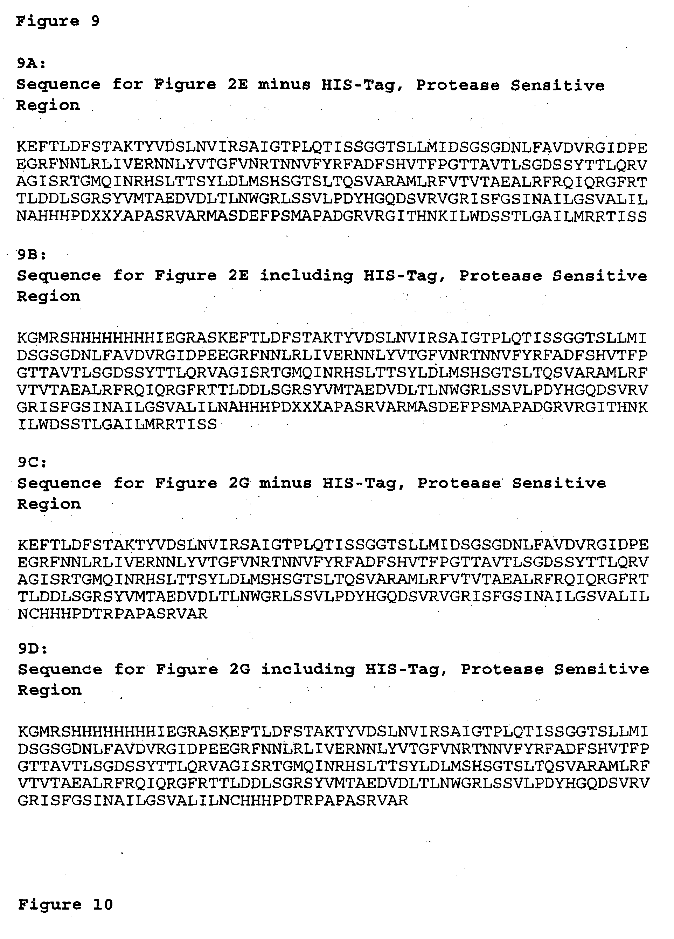

Figure 9A shows the amino acid sequence corresponding to the SLT-1 A chain tripeptide library of Figure 2E, less the N-terminus tags (the His-tag and the protease cleavage site).
Figure 9B shows the amino acid sequence corresponding to the SLT-1 A chain tripeptide library of Figure 2E, with an example of one possible N-terminus tag, comprising a 8-His-tag and a protease cleavage site.
Figure 9C shows the amino acid sequence corresponding to the SLT-1 A1 chain of Figure 2G, less the N-terminus tags (the His-tag and the protease cleavage site).
Figure 9D shows the amino acid sequence corresponding to the SLT-1 A1 chain of Figure 2G, with an example of one possible N-terminus tag, comprising a 8-His-tag and a protease cleavage site.

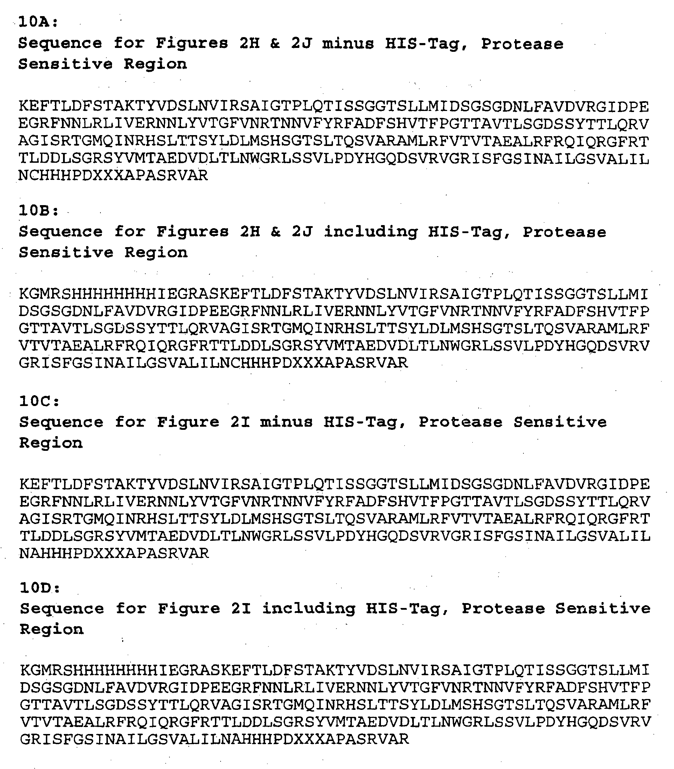

Figure 10A shows the amino acid sequence corresponding to the SLT-1 A1 chain of figure 2H and 2J, less the N-terminus tags (the His-tag and the protease cleavage site).
Figure 10B shows the amino acid sequence corresponding to the SLT-1 A1 chain of figure 2H and 2J, with an example of one possible N-terminus tag, comprising a 8-His-tag and a protease cleavage site.
Figure 10C shows the amino acid sequence corresponding to the SLT-1 A1 chain of figure 2I, less the N-terminus tags (the His-tag and the protease cleavage site).
Figure 10D shows the amino acid sequence corresponding to the SLT-1 A1 chain of figure 2I, with an example of one possible N-terminus tag, comprising a 8-His-tag and a protease cleavage site.
Figure 11A shows the amino acid sequence corresponding to the SLT-1 A chain of Figure 4A, less the N-terminus tags (the His-tag and the protease cleavage site).
Figure 11B shows the amino acid sequence corresponding to the SLT-1 A chain of Figure 4A, with an example of one possible N-terminus tag, comprising a 8-His-tag and a protease cleavage site.
Figure 11C shows the amino acid sequence corresponding to the SLT-1 A chain of Figure 4B and 4E, less the N-terminus tags (the His-tag and the protease cleavage site).
Figure 11D shows the amino acid sequence corresponding to the SLT-1 A chain of Figure 4B and 4E, with an example of one possible N-terminus tag, comprising a 8-His-tag and a protease cleavage site.
Figure 12A shows the amino acid sequence corresponding to the SLT-1 A chain of Figure 4C, less the N-terminus tags (the His-tag and the protease cleavage site).
Figure 12B shows the amino acid sequence corresponding to the SLT-1 A chain of Figure 4C, with an example of one possible N-terminus tag, comprising a 8-His-tag and a protease cleavage site.
Figure 12C shows the amino acid sequence corresponding to the SLT-1 A chain of Figure 4D, less the N-terminus tags (the His-tag and the protease cleavage site).
Figure 12D shows the amino acid sequence corresponding to the SLT-1 A chain of Figure 4D, with an example of one possible N-terminus tag, comprising a 8-His-tag and a protease cleavage site.
Figure 13A shows the amino acid sequence corresponding to the SLT-1 A1 chain of Figure 4F and 4G, less the N-terminus tags (the His-tag and the protease cleavage site).

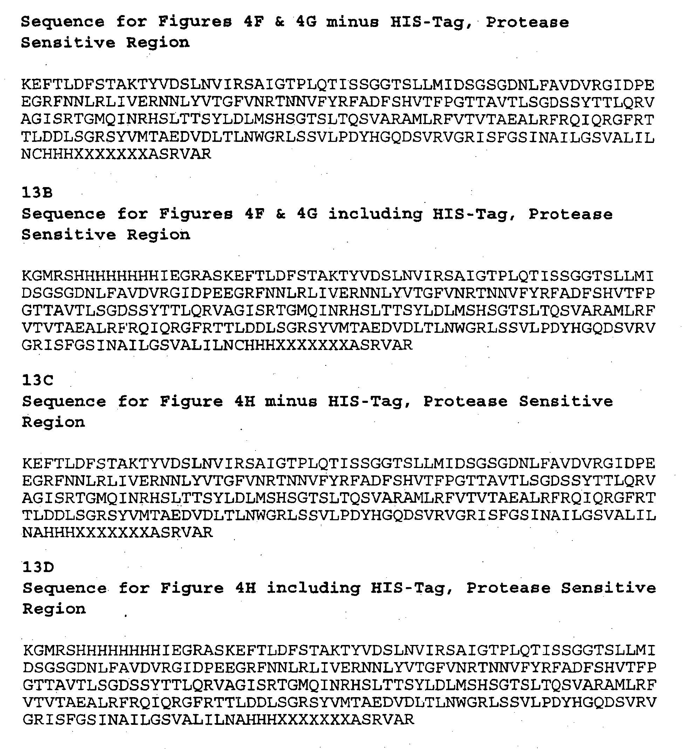

Figure 13B shows the amino acid sequence corresponding to the SLT-1 A1 chain of Figure 4F and 4G, with an example of one possible N-terminus tag, comprising a 8-His-tag and a protease cleavage site.
Figure 13C shows the amino acid sequence corresponding to the SLT-1 A1 chain of Figure 4H, less the N-terminus tags (the His-tag and the protease cleavage site).
Figure 13D shows the amino acid sequence corresponding to the SLT-1 A1 chain of Figure 4H, with an example of one possible N-terminus tag, comprising a 8-His-tag and a protease cleavage site.

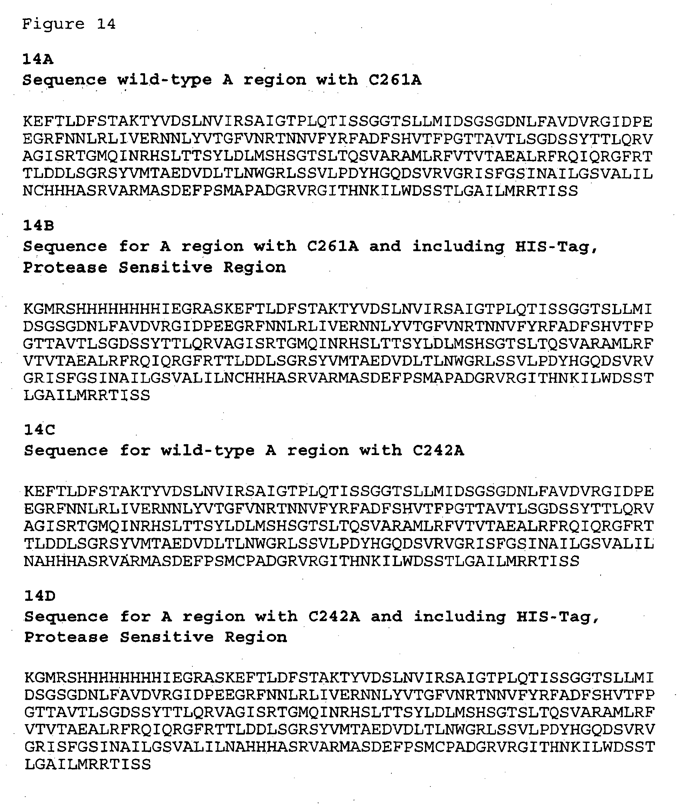

Figure 14A shows the amino acid sequence corresponding to the SLT-1 A region, with cysteine 261 mutated to an alanine.
Figure 14B shows the amino acid sequence corresponding to the SLT-1 A region, with cysteine 261 mutated to an alanine, and with an example of one possible N-terminus tag, comprising an 8-HIS-tag and a protease cleavage site.
Figure 14C shows the amino acid sequence corresponding to the SLT-1 A region, with cysteine 242 mutated to an alanine.
Figure 14D shows the amino acid sequence corresponding to the SLT-1 A region, with cysteine 242 mutated to an alanine, and with an example of one possible N-terminus tag, comprising an 8-HIS-tag and a protease cleavage site.

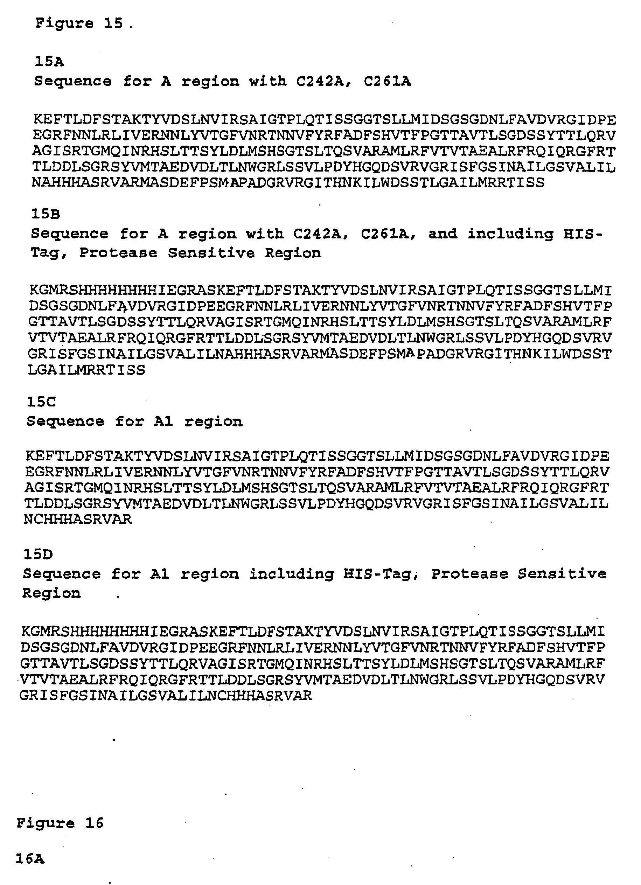

Figure 15A shows the amino acid sequence corresponding to the SLT-1 A region, with cysteines 242 and 261 mutated to alanines.
Figure 15B shows the amino acid sequence corresponding to the SLT-1 A region, with cysteines 242 and 261 mutated to alanines, and with an example of one possible N-terminus tag, comprising an 8-HIS-tag and a protease cleavage site.
Figure 15C shows the amino acid sequence corresponding to the SLT-1 A1 region.
Figure 15D shows the amino acid sequence corresponding to the SLT-1 A1 region with an example of one possible N-terminus tag, comprising an 8-HIS-tag and a protease cleavage site.
Figure 16A shows the amino acid sequence corresponding to the SLT-1 A1 region, with cysteine 242 mutated to an alanine.

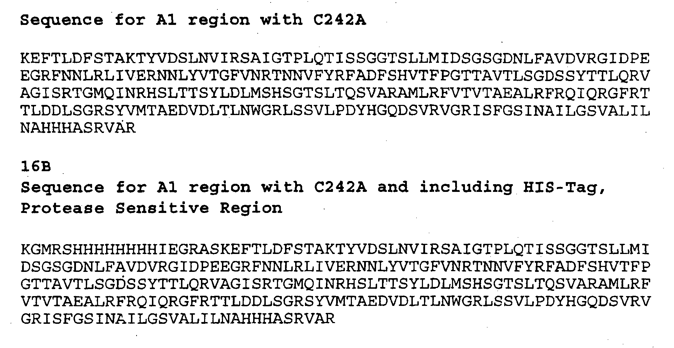

Figure 16B shows the amino acid sequence corresponding to the SLT-1 A1 region with cysteine 242 mutated to an alanine, and with an example of one possible N-terminus tag, comprising an 8-HIS-tag and a protease cleavage site.
For figures 6-16 that include the N-terminus tag, the tag can be varied as needed; for example, the tag KGMRSHHHHHHHHRVARAS can be used instead of KGMRSHHHHHHHHIEGRAS.

### Detailed Description of the Invention

The present invention relates to a combinatorial protein library as defmed in the claims comprising a plurality of protein species, each protein species comprising an A chain of a Shiga-like toxin 1 protein into which an insert has been introduced. The insert is a polypeptide of varying amino acid sequence having a length of from 3 to 200 amino acid residues; and is introduced into the protease-sensitive loop of the A chain sequence. The library provides a collection of protein species that can be screened for individual proteins that are toxic against specific cell types, such as specific cancer cell types. Individual protein species thus selected are suitably used in the treatment of the cancer.

As used in the specification and claims of this application, the term "combinatorial library" refers to a mixture of species each of which has a common portion and a variable portion. In the case of a "combinatorial protein library" each of the species is a protein or peptide, and the common portions and the variable portions are each amino acid sequences. In the case of a combinatorial expression library, the species are microorganisms, expression vectors or polynucleotides which, when expressed, produce proteins or peptides having common portions and variable portions. In this case, the common portions and the variable portions are each nucleotide sequences. Since the purpose of the combinatorial library is to provide multiple variants for screening purposes, the combinatorial library preferably contains at least 100 distinct species of protein or expression unit, more preferably at least 1000 distinct species.

As used in the specification, the term "heteromeric toxic protein" refers to the class of protein toxins with the common organization theme of being heteromeric in nature with two or more polypeptide domains or subunits responsible for distinct functions (Merritt, E.A., and Hol, W.G.J. (1995) Curr. Opin. Struct. Biol. 5:165 1). In such proteins, the two or more subunits or domains could be referred to as A and B, and the toxins as ABₓ toxins where x represents the number of identical or homologous B subunits in the toxin. This family of framework-related toxins includes examples such as Shiga and Shiga-like toxins, the E. coli heat-labile enterotoxins, cholera toxin, diphtheria toxin, pertussis toxin, Pseudomonas aeruginosa exotoxin-A as well as plant toxins such as ricin and abrin. Based on their ability to block protein synthesis, proteins such as Shiga and Shiga-like toxins as well as ricin, abrin, gelonin, crotin, pokeweed antiviral protein, saporin, momordin, modeccin, sarcin, diphtheria toxin and exotoxin A have been referred to as ribosome-inactivating proteins (RIP). In these naturally-occurring heteromeric toxic proteins, the A chain is the toxic portion, while the B chains form a binding moiety which binds to a receptor on a cell susceptible to the toxin, thereby delivering the A chain to the cell. In many cases, the A chain has been further characterized as having two domains, an A1 domain and an A2 domain. Typically, the A1 domain includes the cytotoxic elements of the A subunit and a portion of the protease-sensitive loop portion, but often does not include the B chain binding portion.

One specific example of the A chain of a heteromeric toxic protein is the A chain of SLT-1 which has the sequence given is Seq. ID No. 1. The A chain of SLT-1 comprises of 293 amino acids with the enzymatic (toxic) domain spanning residues 1 to 239. A protease sensitive loop encompassing residues 242 to 261 is normally exposed, and is a suitable site for inserting a peptide sequence. The A chain of SLT-1 is characterized as having an A1 domain, spanning residues 1 to 251, and including the entire enzymatic (toxic) domain, and an A2 domain spanning residues 252-261.

SLT-1 is a type II ribosome inactivating protein produced by pathogenic strain of Escherichia coli (0157:H7) (24). SLT-1 is an AB5 complex of about 70 kD (O'Brien, A. D., and Holmes, R. K. (1987) Shiga and Shiga-like toxins. Microbiol Rev 51, 206-220.). The single 32 kD catalytic A subunit is non-covalently associated with a pentamer of five identical 7.7 kD B subunits. The B subunit pentamer recognizes the glycolipid globotriaosylceramide (also known as CD77 or Gb3) on the surface of target cells (Lingwood, C. A. (1993) Verotoxins and their glycolipid receptors. Adv Lipid Res 25, 189-211; Jacewicz, et al. (1986) Pathogenesis of shigella diarrhea. XI. Isolation of a shigella toxin-binding glycolipid from rabbit jejunum and HeLa cells and its identification as globotriaosylceramide. J Exp Med 163, 1391-1404). A protease-sensitive loop located between Cys242 and 261 at the C terminus of the A chain is cleaved by furin during cellular routing (Fig. 1). The A chain remains associated with its B subunit pentamer due to an intrachain disulfide bond between Cys242 and Cys261 as it travels to the ER lumen (Sandvig, et al. (1989) Endocytosis from coated pits of Shiga toxin: a glycolipid-binding protein from Shigella dysenteriae 1. J Cell Biol 108, 1331-1343;32. Garred, et al. (1995) Role of processing and intracellular transport for optimal toxicity of Shiga toxin and toxin mutants. Exp Cell Res 218, 39-49.). The disulfide bond is finally reduced in the ER lumen and the A1 chain (first 251 aa) is released and subsequently retrotranslocated to the cytosol where it inactivates ribosomes (O'Brien, et al. (1992) Shiga toxin: biochemistry, genetics, mode of action, and role in pathogenesis. Curr Top Microbiol Immunol 180, 65-94.). More specifically, the A chain of SLT-1 is a N-glycosidase that catalytically cleaves a specific adenine nucleotide (4324) from the 28 S rRNA (Brigotti, et al. (1997) The RNA-N-glycosidase activity of Shiga-like toxin I: kinetic parameters of the native and activated toxin. Toxicon 35, 1431-1437.). This event leads to the inhibition of protein synthesis by preventing the binding of aminoacyl tRNAs to the ribosome and halting protein elongation. Mutagenesis studies as well as structural analysis performed on the A chains of ST and ricin have delineated key conserved residues involved in catalytic activity (Deresiewicz, et al.(1992) Mutations affecting the activity of the Shiga-like toxin I A-chain. Biochemistry 31; 3272-3280; Ready, et al. (1991) Site-directed mutagenesis of ricin A-chain and implications for the mechanism of action. Proteins 10, 270-278). Residues important for catalytic activity of SLT-1 are tyrosine 77, glutamic acid 167, arginine 170 and tryptophan 203 (Hovde, et al. (1988) Evidence that glutamic acid 167 is an active-site residue of Shiga-like toxin I. Proc Natl Acad Sci U S A 85,2568-2572; Yamasaki, et al. (1991) Importance of arginine at position 170 of the A subunit of Vero toxin 1 produced by enterohemorrhagic Escherichia coli for toxin activity. Microb Pathog 11, 1-9). In addition, binding of the toxin to the cell surface is important to introduction into the cell and thus for toxic activity. Because of this the A chain alone is not significantly toxic.

In addition to the A or A1 chain of SLT-1, other toxins may also be used to form libraries and compositions described herein, and may be used in the methods described herein. Specifically, Shiga and other Shiga-like toxins, as well as ricin, abrin, gelonin, crotin, pokeweed antiviral protein, saporin, momordin, modeccin, sarcin, diphtheria toxin and exotoxin A, and other functionally-related ribosome inactivating proteins (RIP) are described herein.

For purposes of making the combinatorial library of the invention, short amino acid sequences of 3 to 200 amino acids residues in length, are inserted into this protease sensitive loop. The number of amino acids in the insert defines the number of possible random variants that can be in the library. For example, when the number of amino acids in the insert is 3, the maximum number of variants is 20³ or 8000 variants. Larger inserts provide corresponding larger numbers of possible variants. It has also been found that certain other regions of the A or A1 chain may be used to insert the short amino acid sequences. The present inventors have shown, for example, that the N terminus region can support insertions of long amino acid sequences without significant loss of activity.

As an alternative to using inserts with purely random sequences, inserts can be designed based on a known template. For example, as described below in the context of Muc-1, variations in a sequence known to provide receptor binding properties for a particular cell type can be used to identify an insert that provides for optimization of the toxic properties of the protein construct. This same optimization may be performed on an individual sequence isolated by screening of a larger combinatorial library. It will be appreciated, however, that the insert in the proof of principle tests of Example 1 use an insert which is the target/receptor, while in the actual case the insert would be based on the sequence of a known ligand to be optimized for maximum effectiveness and specificity.

This approach, in which a specific, known receptor type is targeted illustrates a further aspect described herein, namely a method for identifying peptide ligands that bind to specific targets/receptors, such as tumor markers known to exist on cancer cells. In this method, a combinatorial protein library in accordance with the invention is screened against cells known to possess the target/receptor. The toxin serves as the reporter, such that proteins which are shown to be toxic to the cells are evaluated to determine the sequence of the inserted region. Peptides of this sequence can then be used, in combination with a toxin or other molecules, to direct compounds to cells possessing the target/receptor. Further testing peptides of the sequence of the inserted region may be appropriate to confirm that they are a ligand for the specific target/receptor, as opposed to some other receptor on the cell type. This can be done using binding assays with isolated receptor, where such are available.

These inserts used in alternative to inserts with purely random sequences can use, as a known template, a small protein molecule with a known or desirable folded structure, for increasing sensitivity or specificity of binding. For example, an immunoglobulin like domain, such as a FC molecule sequence, a fragment of an FC molecule sequence, or a mimic of an FC molecule sequence, can be inserted. Libraries can therefore be created with a variable chain domain region of an FC molecule, or a random 7-mer (or other length random sequence) inserted in the variable region of an FC molecule, in turn inserted in the protease-sensitive loop region of the A molecule.

The invention also provides a method, as disclosed in the claims for identifying toxic substances specific for a known cell marker, and particularly markers that are available in isolated form. In this embodiment of the invention, the toxin need not serve as reporter. Thus, cells having the marker, or an isolated target/receptor, where available, are exposed to the combinatorial protein library. In preferred embodiments, the cells or the isolated target/receptor are immobilized on a solid support, such as in plastic wells. Captured proteins from the library are then rescreened against cells to confirm their toxicity and specificity for cells expressing the target/receptor, and their suitability for use as a therapeutic. This method can be used to identify toxins with binding inserts specific for any tumor marker or cell receptor, including without limitation tumor markers such as mucins such as MUC-1 and its glycoforms, Her-2, Her2-Neu, tyrosine kinase markers, EGFR, GD2, and GD3.

Thus, in accordance with this specific aspect of the invention, a method for isolating a toxin specific for a known target/receptor as defined in the claims, and comprises the steps of:
(a) exposing the target/receptor to a combinatorial protein library as defined in the claims, comprising a plurality of protein species, each protein species comprising an A chain of a Shiga-like toxin 1 protein into which an insert has been introduced, wherein,
   the insert is a polypeptide of varying amino acid sequence having a length of 3-200 amino acid residues; and
   the insert is introduced into the protease-sensitive loop of the A chain sequence; and
(b) isolating at least one protein species from the combinatorial protein library captured by binding to the target/receptor. As used in the specification and claims hereof, the term "isolating" refers to any mechanism for obtaining a composition containing the protein separated from the milieu in which it is expressed in a form suitable for further analysis. This would include release from the target/receptor following capture (for example by exposure to a competitive binding agent) or isolation from a culture of a clone expressing the protein found to be captured. The method may further comprises the step of screening the isolated protein against cells expressing the target/receptor, to confirm their toxicity for cells expressing the target/receptor. Procedures suitable for this screening are described in Example 3. As noted above, in this method, the target/receptor may be a purified target/receptor and may be immobilized on a solid support. The target/receptor may also be on the surface of cells, which may be immobilized. Where the target/receptor is on the surface of cells, the toxin can serve as a reporter, and the death of the cells is indicative of receptor binding.

Our experience with constructing SLT-1 libraries has pointed out a number of practical issues that are appropriately considered in selecting a protein template for building combinatorial libraries. An important factor is to choose a single chain protein, preferably a bacterial protein of less than 300 amino acids (if the libraries are to be expressed in prokaryotes). Use of a smaller toxin both increases its potential to penetrate into solid tumors and reduces its immunogenicity. Secondly, the protein template should spontaneously fold in solution into its active form such that there is minimal need for host's chaperones. One should avoid for example scaffolds that contain multiple cysteine residues normally involved in disulfide bridges. In addition, a single chain protein as opposed to a multi-subunit complex may be more easily exported from bacteria. Thirdly, the protein template should possess an enzymatic activity, which can rapidly be measured to confirm the proper folding of peptide variants containing single and multiple site-directed mutations. Fourthly, a simple screening approach should be incorporated into the design of combinatorial libraries. Such searches should be amenable to high-throughput screening methods. The catalytic A chain of SLT-1 I (residues 1 to 293) and the A1 chain of SLT-1 (residues 1-251) meet these criteria because they are a single chain that lack any known receptor binding function, and that have well defined structures and catalytic sites.

We have also found that cysteine **242** and 261 of the A chain of SLT-1 can be replaced by site specific mutagenesis (or otherwise modified) in order to remove the single disulfide bridge present between these two cysteines in the A chain. Such advantageous mutations remove the need to reduce the peptide chain during purification and provide a simpler strategy for purifying single chain toxins comprising the A1 domain of A chain mutants. We have found that either cysteine 242 or cysteine 261, or both, can be mutated in this manner. The cysteine(s) can be mutated to alanine, though other mutations may result in the same desired effect while providing a suitable A or A1 chain.

An other advantage of mutating the cysteines in this manner results from the mutation of either of C242 or C261 (preferably, C261). This results in a shiga-like protein A chain with only one cysteine. The thiol group associated with this single cysteine residue-its therefore free and offers the opportunity to introduce spectroscopic (derivatives of fluorescent chromophores that react with the thiol moiety), radioactive (thiol-reactive heavy ion complexes or metal chelators), or biochemical (for example, biotin) probe. These A chain variants, labeled with reporter probes, can then be used as agents for both in vitro and in vivo applications. For example, an inactivated single chain A or A1 variant, labeled with a reporter probe (and inactivated by mutating E 167A and R170A) can be used as a non-toxic probe, as described above, to determine which patient population may benefit from being treated with which of the corresponding catalytically active single chain variants. A library of inactivated single chain A1 variants labeled in this manner can also be used to determine which binding domains (from said library) are effective, to determine which binding domains can be used in corresponding catalytically active single chain variants to treat a patient. Alternatively, these non-toxic probes can be used to determine which binding domains (from the randomly mutated library) can be used to target the desired cell population and used in either the toxic subunit, or bound to a separate and known therapeutic agent, to target and treat that cell population. Alternatively, once a specific desired library member has been identified, either through binding studies as described above or otherwise, that one specific library member (for example, an A1 chain with a specific desirable 7mer sequence inserted after amino acid 245) can be biotinylated for further purification or other studies.

Alternatively, the entire library can be biotinylated, allowing the library to be assayed with a receptor based assay rather than a toxin based assay.

We have also surprisingly found that the use of the A1 domain of the A chain is sufficient for performing the assays described herein. Although the A1 domain contains the entirety of the cytotoxic domain of the A subunit, it was not known until know what function was served by the A2 subunit in assays of this kind, and whether the A1 subunit could be succesfully used on its own in this type of assay. By removing the A2 portion of the A chain, a smaller, more easily manipulated fragment can be used in the assays described herein.

Combining the unexpected finding that cysteine mutations result in a functional A subunit useful in the assays described herein, with the finding that the A1 subunit can be used alone yields the further advantage of combining these two findings. By mutating one of the cysteines (for example, C261), one can make a protein that can not form the protease-sensitive A chain loop (since this protease-sensitive A chain loop is a result of the cysteine-cysteine interactions between C242 and C261). The A subunit can therefore be cleaved more efficiently into A1 and A2 subunits. Thus mutation of at least one of C242 and C261, combined with utilizing the A1 subunit alone, provides a simpler and more elegant strategy for purifying single chain toxins comprising the A1 domain of A chain mutants. Manufacturing, and purifying, a protein or a protein library of use in the assays described herein thus becomes much more efficient by either utilizing the A1 domain alone, at least one cysteine mutation, or both.

A further aspect described herein is a combinatorial expression library comprising a plurality of species of expression systems. Each species within the expression library expresses a protein species comprising an A or A1 chain of a heteromeric toxic protein into which an insert has been introduced. The insert is a polypeptide of varying amino acid sequence having a length of 2 or more amino acid residues, for example from 3 to 200 amino acid residues; and is introduced into the protease-sensitive loop of the A chain sequence. Suitable expression systems include plasmids and viral vectors.

The A chain can be pegylated or otherwise modified by a method known in the art in order to stabilize or modify the biological half-life of the protein. Reactive pegylation agents can be bound to a lysine, to the N-terminus, or to cysteine 242 or 261.

We have also found that adding a histidine tag (for example, a 6 or 8 histidine tag) to the N-terminus of the A or A1 chain is advantageous as it allows for improved purification of the desired protein. Optionally, a protease-sensitive region can be inserted between the histidine tag and the rest of the A or A1 chain in order to facilitate cleavage of the histidine tag from the A or A1 chain once it is no longer desired. Examples of protease-sensitive regions that may be used include a thrombin sensitive region or a furin sensitive region. Other protease-sensitive sequences are also well known in the art.

The invention will now be further described with reference to the following, nonlimiting examples.

### Example 1

### Proof-of-concept: design and mining of a prototypic A chain-tripeptide library

We originally created a simple tripeptide library inserted in the C-terminal protease-sensitive loop of the SLT-1 A chain (Figs. 2A and B). This A chain loop region is naturally constrained due to the presence of a single disulfide bond bridging Cys242 to Cys261. The maximal diversity of this library can thus be calculated to be 20³ or 8000 permutations of a tripeptide sequence. As a proof-of-concept that A chain libraries can easily be screened for a new receptor-binding activity, we picked more than 3000 colonies from this A chain-tripeptide library and purified the mutant toxin produced by each clone. We noticed very early in this study that the level of expression of A chain mutant was dramatically increased when expressed in the presence of the wild-type SLT-1 B subunit. Thus the mutant forms of A chain were expressed and initially purified as AB5 toxin variants. Since all A subunits harbor a polyHis purification tag, it is relatively easy to remove the B subunit with denaturants (urea for example) while recovering the A chain on metal-affinity columns or beads. Western blots performed on randomly selected bacterial clones indicated that > 70% of these colonies produced significant amounts of these A chain mutants.

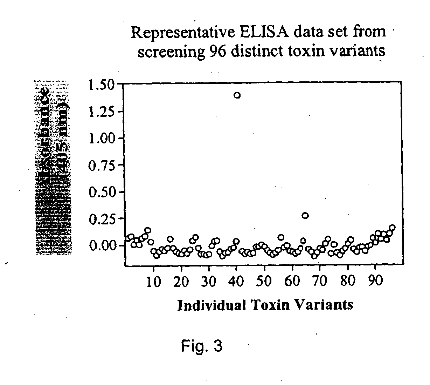

These toxin variants were then coated in individual wells of 96-well plates and screened by ELISA for their ability to bind the monoclonal antibody Onc M27 (Linsley, et al.(1988) Monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). As shown in Tables 1 and 2, most A chain mutants neither coded for the tripeptide insert that matched the targeted epitope of Onc M27 nor were recognized by the antibody. However on two occasions, a toxin variant harboring the exact epitope (Table 1, Fig.3) gave a strong ELISA signal comparable to the one observed with our control A chain harboring the MUC 1 tripeptide epitope and had the expected epitope sequence in its randomized tripeptide region. A typical ELISA data set for 96 A chain mutants is presented in Fig. 3, highlighting the fact that the majority of A chain mutants did not recognized the mAb Onc M27 except for an A chain variant (mutant #41 in Tables 1 and 2). These results clearly established that A chain libraries can easily be constructed and screened to find toxin variants able to specifically target a given receptor, in this case an antigen-combining site.

### Example 2

### Proof-of-concept: design and mining of a prototypic A1 chain-tripeptide library

A tripeptide library inserted in the C-terminal SLT-1 A1 domain is prepared. A simple tripeptide library is inserted in the C-terminal protease-sensitive loop of the SLT-1 A chain (Figs. 2A and B). This A chain loop region is naturally constrained due to the presence of a single disulfide bond bridging Cys242 to Cys261. The maximal diversity of this library can thus be calculated to be 20³ or 8000 permutations of a tripeptide sequence. A chain libraries can easily be screened for a new receptor-binding activity. Over 3000 colonies from this A chain tripeptide library are picked and the mutant toxin produced by each clone is purified. The level of expression of A chain mutant is dramatically increased when expressed in the presence of the wild-type SLT-1 B subunit. Thus the mutant forms of A chain are expressed and initially purified as AB5 toxin variants. Since all A subunits harbor a polyHis purification tag, it is relatively easy to remove the B subunit with denaturants (urea for example) while recovering the A chain on metal-affinity columns or beads. Western blots performed on randomly selected bacterial clones indicate that > 70% of these colonies produce significant amounts of these A chain mutants. The A1 subunit is then excised from the A chain mutants, using a protease cleavage using furin (Fig. 2G and H).

These toxin variants are then coated in individual wells of 96-well plates and screened by ELISA for their ability to bind the monoclonal antibody Onc M27 (Linsley, et al.(1988) monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC 1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibody SM-3 is selectively exposed in a range of primary carcinomas. Int J Cancer 43, 1072-1076). Most A1 chain mutants neither code for the tripeptide insert that match the targeted epitope of Onc M27 nor are recognized by the antibody. However on a few occasions, a toxin variant harboring the exact epitope gives a strong ELISA signal comparable to the one observed with our control A chain harboring the MUC1 tripeptide epitope and has the expected epitope sequence in its randomized tripeptide region. These results clearly establish that A1 chain libraries can easily be constructed and screened to find toxin variants able to specifically target a given receptor, in this case an antigen-combining site.

### Example 3

### Proof-of-concept: Alternative method for design and mining of a prototypic A1 chain-tripeptide library

A tripeptide library inserted in the C-terminal SLT-1 A1 domain is prepared. A simple tripeptide library is inserted in the C-terminal protease-sensitive loop of the SLT-1 A1 chain (Fig. 2 G and H). This A1 domain codes for A region amino acids 1-251. The maximal diversity of this library can thus be calculated to be 20³ or 8000 permutations of a tripeptide sequence. A chain libraries can easily be screened for a new receptor-binding activity. Over 3000 colonies from this A1 chain tripeptide library are picked and the mutant toxin produced by each clone is purified. Since all A1 subunits lack a B subunit binding area, it is relatively easy to exise any B subunits. Optionally, the polyHIS tags can be used to further purify the A1 subunits, as described in previous examples.

These toxin variants are then coated in individual wells of 96-well plates and screened by ELISA for their ability to bind the monoclonal antibody Onc M27 (Linsley, et al.(1988) monoclonal antibodies reactive with mucin glycoproteins found in sera from breast cancer patients. Cancer Res 48, 2138-2148.), directed at the well-characterized breast cancer tripeptide epitope Thr-Arg-Pro of the human MUC1 tandem repeat (Gendler, et al. (1988) A highly immunogenic region of a human polymorphic epithelial mucin expressed by carcinomas is made up of tandem repeats. J Biol Chem 263, 12820-12823; Girling, et al. (1989) A core protein epitope of the polymorphic epithelial mucin detected by the monoclonal antibodySM-3 is selectively exposed in a range of primary carcinomas. Int J Cancer 43, 1072-1076). Most A1 chain mutants neither code for the tripeptide insert that match the targeted epitope of Onc M27 nor are recognized by the antibody. However on a few occasions, a toxin variant harboring the exact epitope gives a strong ELISA signal comparable to the one observed with our control A chain harboring the MUC1 tripeptide epitope and has the expected epitope sequence in its randomized tripeptide region. These results clearly establish that A1 chain libraries can easily be constructed and screened to find toxin variants able to specifically target a given receptor, in this case an antigen-combining site.

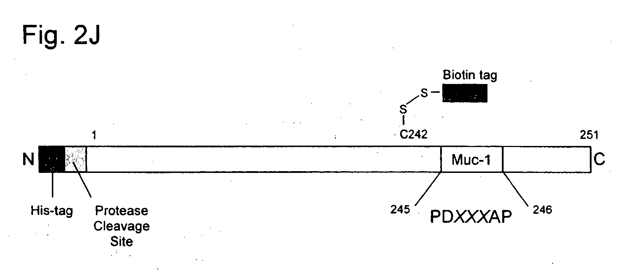

The A1 tripeptide library can also be biotinylated at the C242 position (Figure 2J). A standard biotin assay can then be used to determine whether an A1 tripeptide library member is present, or bound to a substrate or given receptor.

### Example 4

### Proof-of-concept: Method for design and mining of a prototypic A chain-tripeptide library with a mutation in C242 and/or C 261

A tripeptide library inserted in the C-terminal SLT-1 A domain is prepared, using the method of Example 1 with the modification that encoding for the cysteine at position 242 and/or position 261 has been mutated to an alanine using site directed mutagenesis (Figures 2C, 2D, or 2E). For libraries containing one cysteine in the A chain, the tripeptide library is optionally biotinylated (Fig. 2F). The A chain libraries can easily be screened for a new receptor-binding activity. Results indicate that these cys to ala modified A chain libraries are at least as effective as the wild-type libraries of Example 1 in ease of construction and screening to find toxin variants able to specifically target a given receptor. In the case of the biotinylated libraries, the biotin labels are easily used, utilizing known methods, to identify whether a member of the A chain library is present or bound to a sample.

### Example 5

### Proof-of-concept: Method for design and mining of a prototypic A1 chain-tripeptide library with a mutation in C242 and/or C 261

A tripeptide library inserted in the C-terminal SLT-1 A1 domain is prepared, using the method of Example 2 with the modification that encoding for the cysteine at position 242 and/or position 261 has been mutated to an alanine using site directed mutagenesis (Fig. 2I). The A1 chain libraries can easily be screened for a new receptor-binding activity. Results indicate that these cys to ala modified A1 chain libraries are as effective as the wild-type libraries of Example 2 in ease of construction and screening to find toxin variants able to specifically target a given receptor. Results indicate that these cys to ala modified A1 chain libraries are significantly easier to make and purify, due to the lack of the disulfide bridge between Cys 242 and Cys 261, resulting in a much easier cleavage of A2 from A1 in the protease cleavage step. In the case of an A1 tripeptide library having a cysteine at position C242, the cysteine is optionally tagged with biotin (Fig. 2J). This allows for determination of whether the library member is present, or bound to a substrate or receptor, using known methods.

### Example 6

### Proof-of-concept: Method for design and mining of a prototypic A1 chain-tripeptide library with a mutation in C242 and/or C 261

A tripeptide library inserted in the C-terminal SLT-1 A1 domain is prepared, using the method of Example 3 with the modification that encoding for the cysteine at position 242 has been mutated to an alanine using site directed mutagenesis (Fig. 2I). The A1 chain libraries can easily be screened for a new receptor-binding activity. Results indicate that these cys to ala modified A1 chain libraries are as effective as the wild-type libraries of Example 4 in ease of construction and screening to find variants able to specifically target a given receptor.

### Example 7

### Making of Combinatorial SLT-1 A-heptapeptide or A1-heptapeptide Library

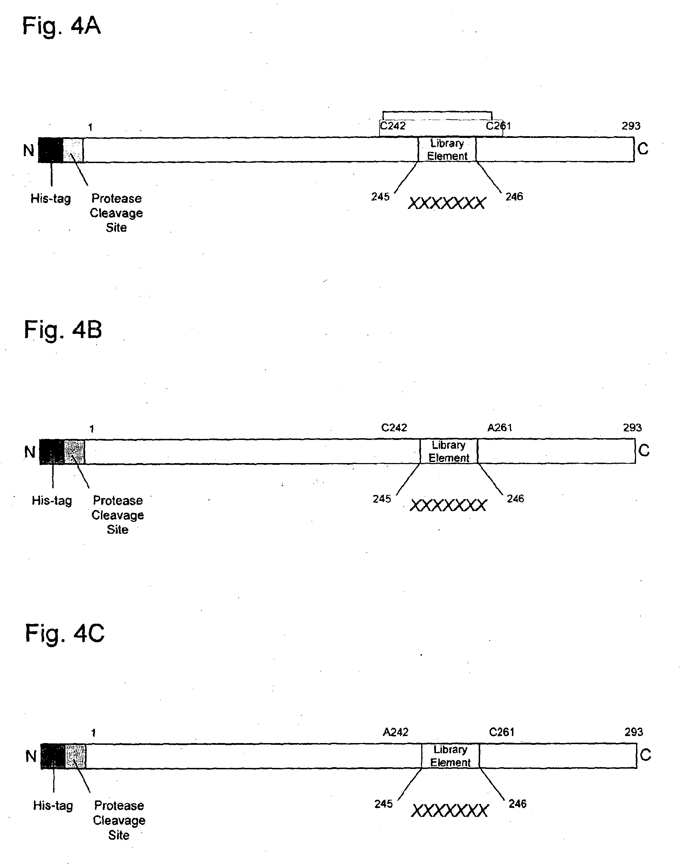

Library diversity represents a crucial parameter in screening combinatorial libraries for ligands able to bind specifically and with high affinity to a particular target. The SLT-1 A chain-tripeptide library described in Examples 1 or 4, and the SLT-1 A1 chain-tripeptide libraries described in Examples 2, 3, 5 and 6 have a maximal diversity of 20³ or 8000 possible mutated A chains. This sampling repertoire is small but was useful in mapping a tripeptide epitope to establish our proof-of-concept. A seven-residue library (20⁷ or 1.3 x 10⁹ possible mutants) represents a more typical minimal diversity level commonly used in designing phage display as well as synthetic peptide libraries. Thus, as a starting point, we build SLT-1 A and A1 chain libraries (as described in Examples 1-6) with a 7-amino acid long random sequence inserted in its C-terminus (Fig. 4A-H). Each of these libraries provide sufficient diversity to insure that A chain or A1 chain toxin variants can be identified that target new or known internalized receptors on cancer cells. All elements of each of these libraries (as well as all other libraries proposed) contain a N-terminal His tag to quickly purify A chain mutants. The libraries (Fig. 4A-4H) are generated using a megaprimer PCR strategy (Sarkar G, and Sommers S, (1990). The 'megaprimer' method of site-directed mutagenesis. Biotechniques 8, 404-407). The megaprimer strategy is widely used to introduce mutations into target DNA sequence, by way of two rounds of PCR that use two flanking primers and an internal mutagenic primer.

All A chain-heptapeptide libraries and A1 chain-heptapeptide libraries are made, as follows. The SLT-1 A chain-heptapeptide libraries (Fig. 4A-4H) harbor a 7-amino acid random insertion between amino acid 245 and 246 of the A or A1 chain, the identical site used to construct our SLT-1 A chain-tripeptide library (Tables 1, 2, Fig. 3). Briefly, construction of the A chain heptapeptide libraries is described as follows using as the example an A chain containing 2 cysteines. Construction of the A1 chain heptapeptide libraries is undertaken using similar methods, with modifications as necessary to form an A1 chain (and as described in parallel Examples 2, 3, 5 and 6, above).

Two flanking primers A (GTT ACT GTG ACA GCT GAA GCT TTA CGT TTT CG (Seq. ID No. 2) and B (GAG AAG AAG AGA CTG CAG ATT CCA TCT GTT G (Seq. ID No. 3)) carrying Hind 111 and Pst1 restriction sites respectively were annealed within the 5' and 3' ends of the SLT-1 operon. A library oligonucleotide F containing all seven random amino acid (NNS) as well as a long matching sequence to anneal to the template were synthesized. In the synthesis of the random oligonucleotide, the relative representation of each amino acid was improved by restricting the third position of each codon to G or T (Noren, K. A., and Noren, C. J. (2001) Construction of high-complexity combinatorial phage display peptide libraries. Methods 23, 169-178.). This type of restriction reduces the overall DNA sequence complexity as well as coding discrepancy between residues (Reidhaar-Olson, et al. (1991) Random mutagenesis of protein sequences using oligonucleotide cassettes. Methods Enzymol 208, 564-586.). This strategy also minimizes the occurrence of stop codons (TAA and TGA) while the stop codon (TAG) is suppressed by using a supE bacterial strain, which specifies for the insertion of a Gln residue when TAG codons are translated. The first PCR reaction was performed using primers A and F and the resulting product purified by denaturing polyacrylamide gel electrophoresis. This product then served as the megaprimer with primer B for a second PCR reaction to amplify the random DNA. The final library DNA (PCR product) will then be digested with Hind 111 and Pst1 and cloned into the backbone of a pECHE9a expression vector (MTI, Toronto). The E. coli strain JM101 was subsequently transformed with the resulting pECHE vector and single bacterial colonies picked, lysed and their supernatants analyzed for the expression of single A chain toxins or layered on cancer cells and screened using the SRB for cell cytotoxicity assay.

### Example 8

### Mining the combinatorial SLT-1 A-heptapeptide library against cancer cell lines using a cytotoxicity assay

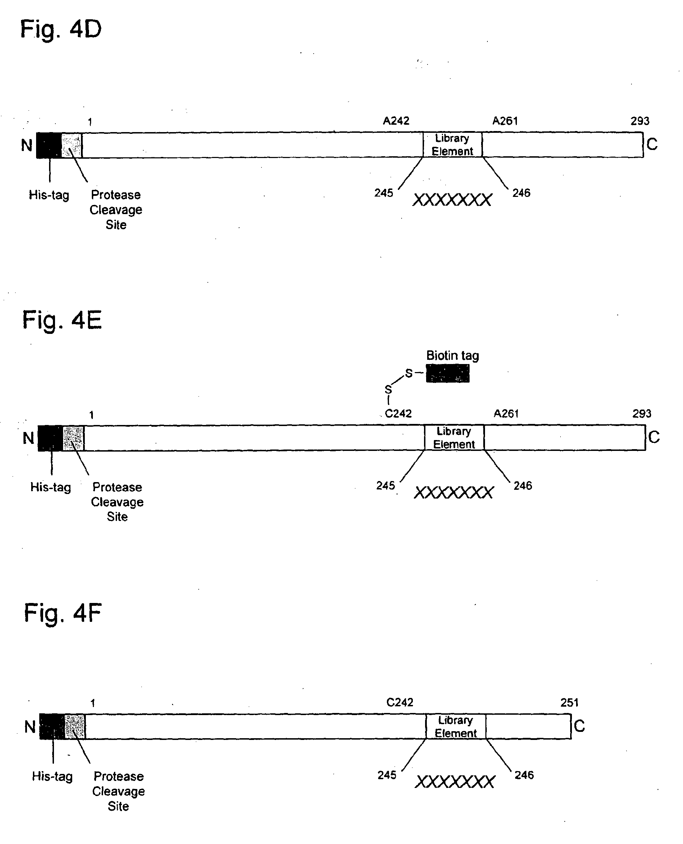

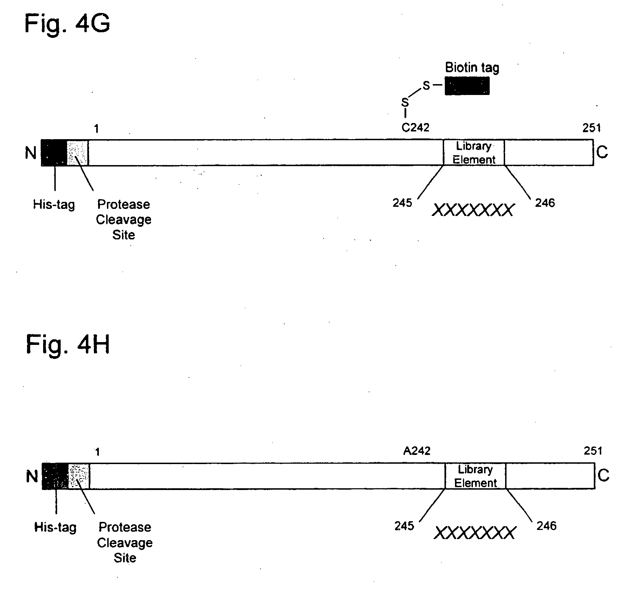

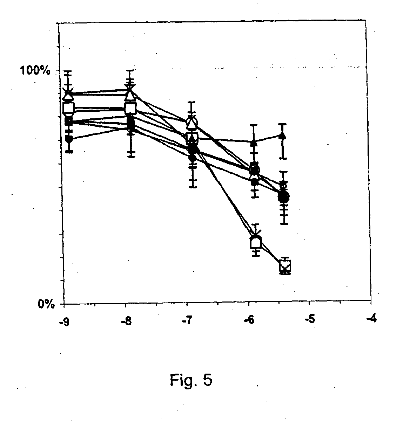

We screen our SLT-1 A-heptapeptide libraries and our SLT-1 A1-heptapeptide libraries using the cytotoxic function of the A or A1 chain as a reporter signal. Cytotoxicity is a more informative property to measure than binding to a receptor since it implies that the toxin is internalized, processed and delivered near ribosomes, clearly a multi-step event. The cytotoxicity assay is essentially performed as previously described (Bray, et al. (2001) Probing the surface of eukaryotic cells using combinatorial toxin libraries. Current Biology 11, 697-701). Briefly, the strategy to screen all our A chain libraries is based on the following principles. Established cancer cell lines such as SK-BR-3 (human breast), CAMA-1 (human breast), 518A2 (human melanoma), PC3 (human prostate) and B16 (murine melanoma) are grown in 96-well plates and used as targets in the primary screen stages. These cell lines are initially selected for our holotoxin library searches (Bray, supra) based on their adherence (plastic), their cell viability staining properties (SRB) in a high-throughput screening setting as well as their lack of receptor and sensitivity to native SLT-1 (to insure a reduced level of false positives). Single bacterial colonies from each library are picked and grown in 96 deep well plates. The cells are harvested, lysed, and their lysates clarified. Since all expressed SLT-1 A chain variants or A1 chain variants have a histidine tag at their N-terminus, each of them is purified from their lysate using nickel-affinity beads (96-well format) and layered on target cells. The plates containing the target cells treated with A chain variants or A1 chain variants are then incubated at 37°C for 48 hours, followed by fixation and staining with Sulforhodamine B (SRB). The SRB assay is a colorimetric end-point assay, which quantifies viable cells by staining their cellular protein content (Skehan, et al. (1990) New colorimetric cytotoxicity assay for anticancer-drug screening. J Natl Cancer Inst 82, 1107-1112.). The SRB assay has been adopted by NCI/NIH for their high-throughput screening of drug candidates on cancer cell lines. Viability assays are repeated for any bacterial extracts leading to cell death. A first round of screening is performed on more than 5000 bacterial clones (equivalent to 5000 distinct A chain toxins) for each of the A chain and A1 chain pentapeptide libraries as described in Figures 4A to 4H, and several toxin variants are identified in each library as repeatable killers of the human melanoma cell line 518A2 (Fig. 5). We find that the A1 chain libraries of figures 4 F, 4 G, and 4 H have similar cytotoxic properties as of that of the A chain libraries. We also find that the libraries with mutations at cysteine 242 or cysteine 261, or at both cysteines, as described in Figures 4B, 4C, 4D, and 4E also have similar cytotoxic properties as that of the "wild type" A chain library of Figure 4A.

The abscissa represents the log concentration of toxin used to treat the cells and the ordinate depicts the observed percentage of cells that are viable after 48 hours. The closed triangles depicts the effect of the wild type toxin on 518A2 cells while the two most efficacious A chain variants were termed SAM#3 (open squares) and SAM#5 (X symbols).

Promising A chain and A1 chain variants from each of the heptapeptide libraries are then re-screened against a panel of cell lines (Vero [Monkey, normal kidney]; PC-3 [Human, prostate cancer]; HepG2 [Human, hepatoma]; SiHa [Human, cervical cancer]; PanC [Human, pancreatic cancer]; SKBR-3 [Human, breast cancer]; 518-A2 [Human, melanoma]; U87 [Human, glioma]; B16-F10 [Mouse, melanoma]; HS-216 [Human, normal fibroblast]; CAMA-1 [Human, breast cancer]; OVCar-3 [Human, ovarian cancer]). Of these variants, a significant proportion are observed to have activity against each of the cancer cell line, the 518-A2 human melanoma, and the SiHa (human cervical cancer cells) and U87-A (human brain cancer cells; glioma) cell lines.

The genes coding for the two A chain toxins (SAM3 and SAM5) that resulted in toxicity of the human melanoma cell lines were sequenced to determine the amino acid sequences inserted between residues 245 and 246 of the wild-type A chain. The sequences, including the His-tag, are listed in Seq. ID Nos 4 and 5, respectively.

**Table 1: DNA sequences of randomly picked clones from the SLT-1 A chain-tripeptide library. Mutated bases in bold characters. Mutant #41 was identified in our ELISA screen as a strong binder of mAb Onc M27 (Fig. 3).**

| SLT-1 A chainNucleotide sequence variant | | Diversity (nucleotide changes in mutated region) |
|---|---|---|
| MUC1 epitope | CCA GAC ACG CGA CCA GCT CCA | 0/9 |
| Mutant #1 | CCA GAC GGG ATC GGG GCT CCA | 8/9 |
| Mutant #2 | CCA GAC CTG GAG ATG GCT CCA | 8/9 |
| Mutant #3 | CCA GAC CCC CGT GGG GCT CCA | 6/9 |
| Mutant #4 | CCA GAC GAC GAC TTG GCT CCA | 9/9 |
| Mutant #5 | CCA GAC GTC CGG TGG GCT CCA | 7/9 |
| Mutant #6 | CCA GAC CAG CGC TGG GCT CCA | 6/9 |
| Mutant #7 | CCA GAC CTC AGG ATG GCT CCA | 8/9 |
| Mutant #8 | CCA GAC TCC CAG GAG GCT CCA | 7/9 |
| Mutant #9 | CCA GAC TCC GAC CCC GCT CCA | 6/9 |
| Mutant #41 | CCA GAC ACG CGC CCC GCT CCA | 2/9 |

**Table 2: Amino acid sequence alignment of randomly selected clones from the SLT-1 A chain-tripeptide library and ELISA signal of purified SLT-1 A chain variants detected with a mAb (Onc M27) raised against the MUC1 epitope Thr-Arg-Pro. Mutated tripeptide region in bold characters. Mutant #41 was identified in our ELISA screen as a strong binder of mAb Onc M27.**

| SLT-1 A chainDeduced amino acid sequence variant | | ELISA readings (405 nm) |
|---|---|---|
| MUC1 epitope | CHHHPD TRP APASRVARMASDEFPSMC | 1.3 |
| Mutant #1 | CHHHPD GIG APASRVARMASDEFPSMC | 0.08 |
| Mutant #2 | CHHHPD LQM APASRVARMASDEFPSMC | 0.03 |
| Mutant #3 | CHHHPD PRG APASRVARMASDEFPSMC | 0.03 |
| Mutant #4 | CHHHPD DDL APASRVARMASDEFPSMC | 0.06 |
| Mutant #5 | CHHHPD VRW APASRVARMASDEFPSMC | 0.07 |
| Mutant #6 | CHHHPD QRL APASRVARMASDEFPSMC | 0.06 |
| Mutant #7 | CHHHPD LRM APASRVARMASDEFPSMC | 0.11 |
| Mutant #8 | CHHHPD SQE APASRVARMASDEFPSMC | 0.13 |
| Mutant #9 | CHHHPD SDP APASRVARMASDEFPSMC | 0.07 |
| Mutant #41 | CHHHPD TRP APASRVARMASDEFPSMC | 1.25 |

## Claims

1. A combinatorial protein library comprising a plurality of protein species, each protein species comprising an A chain of a Shiga-like toxin I protein modified by mutation of amino acid Cys242 and/ or Cys 261 of the sequence shown in Figure 14A, Figure 14C, or Figure 15A, the modified A chain further comprising a protease-sensitive loop as defined with reference to amino acids 242 to 261 of the sequence shown in Figure 14A, Figure 14C, or Figure 15A, into which an insert has been introduced, the insert comprising a polypeptide of varying amino acid sequence having a length of 3 to 200 amino acid residues.

2. The combinatorial protein library of Claim 1, wherein the modified A chain comprises a cysteine residue and further comprises a label or probe bound to the cysteine residue.

3. The combinatorial protein library of Claim 1 or 2, wherein the mutation in at least one cysteine is a mutation from cysteine to an alanine.

4. The combinatorial protein library of any previous claim, wherein the protein species is formed by introducing the insert between amino acids 245 and 246, as defined with reference to the sequence shown in Figure 14A, Figure 14C, or Figure 15A.

5. The combinatorial protein library of any previous claim, wherein the insert has a length of 7 amino acids.

6. A mutant protein comprising an A chain of a Shiga-like toxin I protein modified by mutation of amino acid Cys242 and/ or Cys 261 of the sequence shown in Figure 14A, Figure 14C, or Figure 15A, the modified A chain further comprising a protease-sensitive loop as defined with reference to amino acids 242 to 261 of the sequence shown in Figure 14A, Figure 14C, or Figure 15A, into which an insert has been introduced, the insert comprising a polypeptide of varying amino acid sequence having a length of 3 to 200 amino acid residues.

7. The mutant protein of Claim 6, wherein the mutation in at least one cysteine is a mutation from cysteine to an alanine.

8. The mutant protein of Claim 6, wherein the insert is introduced between amino acids 245 and 246, as defined with reference to the sequence shown in Figure 14A, Figure 14C, or Figure 15A.

9. The mutant protein of Claim 8, wherein the insert comprises the sequence IYSNKLM.

10. The mutant protein of Claim 8, wherein the insert comprises the sequence AAFADLI.

11. A method for isolating a toxin specific for a target/receptor comprising the steps of:
(a) exposing the target/ receptor to a combinatorial protein library according to any of Claims 1 to 5; and
(b) isolating at least one protein species from the combinatorial protein library captured by binding to the target/ receptor.

12. The method of Claim 11, further comprising the step of screening the isolated protein against cells expressing the target/ receptor, to confirm their toxicity for cells expressing the target/receptor.

13. The method of Claim 11, wherein the target/ receptor is a purified target/ receptor and is immobilized on a solid support.

14. The method of Claim 11, wherein the target/receptor is on the surface of cells.

15. The method of Claim 14, wherein the cells are immobilized on a solid support.

16. The method of any of Claims 12 to 15, wherein the toxin serves as a reporter, and the death of the cells is indicative of receptor binding.

17. The method of any of Claims 12 to 15, wherein the cells are cancer cells.

18. The combinatorial protein library of Claim 2, wherein the modified A chain is a modified A chain of Shiga-like toxin I, and wherein the labels or probes are selected from a group consisting of spectroscopic, radioactive, and biochemical labels or probes.

19. A combinatorial protein library comprising a plurality of protein species, each protein species comprising an A chain of a Shiga-like toxin I protein modified by mutation of amino acid Cys242 and/ or Cys 261 pf the sequence shown in Figure 14A, Figure 14C, or Figure 15A, the modified A chain further comprising a protease-sensitive loop as defined with reference to amino acids 242 to 261 of the sequence shown in Figure 14A, Figure 14C, or Figure 15A, into which an insert has been introduced, the insert consisting of a variable domain sequence of an Fc molecule.

## Patentansprüche

1. Kombinatorische Proteinbibliothek, die eine Vielzahl von Proteinarten umfasst, wobei jede Proteinart eine A-Kette eines Shiga-artigen Toxin-I-Proteins umfasst, das durch Mutation der Aminosäure Cys242 und/oder Cys 261 der Sequenz modifiziert ist, die in Figur 14A, 14C oder 15A gezeigt ist, wobei die modifizierte A-Kette ferner eine Protease-sensitive Schleife umfasst, die unter Bezugnahme auf die Aminosäuren 242 bis 261 der Sequenz definiert ist, die in Figur 14A, 14C oder 15A gezeigt ist und in die ein Einsatz eingeführt wurde, wobei der Einsatz ein Polypeptid mit einer variierenden Aminosäuresequenz mit einer Länge von 3 bis 200 Aminosäureresten umfasst.

2. Kombinatorische Proteinbibliothek nach Anspruch 1, wobei die modifizierte A-Kette einen Cysteinrest umfasst und ferner ein Etikett oder eine Sonde umfasst, die an den Cysteinrest gebunden ist.

3. Kombinatorische Proteinbibliothek nach Anspruch 1 oder 2, wobei die Mutation in mindestens ein Cystein eine Mutation von Cystein zu Alanin ist.

4. Kombinatorische Proteinbibliothek nach einem der vorhergehenden Ansprüche, wobei die Proteinart durch Einführen des Einsatzes zwischen die Aminosäuren 245 und 246 gebildet wird, wie unter Bezugnahme auf die Sequenz definiert, die in Figur 14A, 14C oder 15A gezeigt ist.

5. Kombinatorische Proteinbibliothek nach einem der vorhergehenden Ansprüche, wobei der Einsatz eine Länge von 7 Aminosäuren aufweist.

6. Mutiertes Protein, das eine A-Kette eines Shiga-artigen Toxin-I-Proteins umfasst, das durch Mutation der Aminosäure Cys242 und/oder Cys 261 der Sequenz modifiziert ist, die in Figur 14A, 14C oder 15A gezeigt ist, wobei die modifizierte A-Kette ferner eine Protease-sensitive Schleife umfasst, die unter Bezugnahme auf die Aminosäuren 242 bis 261 der Sequenz definiert ist, die in Figur 14A, 14C oder 15A gezeigt ist und in die ein Einsatz eingeführt wurde, wobei der Einsatz ein Polypeptid mit einer variierenden Aminosäuresequenz mit einer Länge von 3 bis 200 Aminosäureresten umfasst.

7. Mutiertes Protein nach Anspruch 6, wobei die Mutation in mindestens ein Cystein eine Mutation von Cystein zu Alanin ist.

8. Mutiertes Protein nach Anspruch 6, wobei der Einsatz zwischen die Aminosäuren 245 und 246 eingeführt wird, wie unter Bezugnahme auf die Sequenz definiert, die in Figur 14A, 14C oder 15A gezeigt ist.

9. Mutiertes Protein nach Anspruch 8, wobei der Einsatz die Sequenz IYSNKLM umfasst.

10. Mutiertes Protein nach Anspruch 8, wobei der Einsatz die Sequenz AAFADLI umfasst.

11. Verfahren zur Isolierung eines Toxins, das für ein Ziel bzw. einen Rezeptor spezifisch ist, wobei das Verfahren folgende Schritte umfasst:
(a) Aussetzen des Ziels bzw. Rezeptors einer kombinatorischen Proteinbibliothek nach einem der Ansprüche 1 bis 5 und
(b) Isolieren mindestens einer Proteinart von der kombinatorischen Proteinbibliothek, die durch Bindung an das Ziel bzw. den Rezeptor eingefangen wurde.

12. Verfahren nach Anspruch 11, das ferner den Schritt des Überprüfens des isolierten Proteins in Bezug auf Zellen umfasst, die das Ziel bzw. den Rezeptor exprimieren, um ihre Toxizität für Zellen zu bestätigen, die das Ziel bzw. den Rezeptor exprimieren.

13. Verfahren nach Anspruch 11, wobei das Ziel bzw. der Rezeptor ein gereinigtes Ziel bzw. ein gereinigter Rezeptor ist und auf einem festen Träger immobilisiert ist.

14. Verfahren nach Anspruch 11, wobei sich das Ziel bzw. der Rezeptor auf der Oberfläche von Zellen befindet.

15. Verfahren nach Anspruch 14, wobei die Zellen auf einem festen Träger immobilisiert sind.

16. Verfahren nach einem der Ansprüche 12 bis 15, wobei das Toxin als Berichterstattungsmittel dient und der Tod der Zellen eine Rezeptorbindung anzeigt.

17. Verfahren nach einem der Ansprüche 12 bis 15, wobei die Zellen Krebszellen sind.

18. Kombinatorische Proteinbibliothek nach Anspruch 2, wobei die modifizierte A-Kette eine modifizierte A-Kette von Shiga-artigem Toxin I ist, und wobei die Etiketten oder Sonden aus einer Gruppe ausgewählt werden, die aus spektroskopischen, radioaktiven und biochemischen Etiketten oder Sonden besteht.

19. Kombinatorische Proteinbibliothek, die eine Vielzahl von Proteinarten umfasst, wobei jede Proteinart eine A-Kette eines Shiga-artigen Toxin-I-Proteins umfasst, das durch Mutation der Aminosäure Cys242 und/oder Cys 261 der Sequenz modifiziert ist, die in Figur 14A, 14C oder 15A gezeigt ist, wobei die modifizierte A-Kette ferner eine Protease-sensitive Schleife umfasst, die unter Bezugnahme auf die Aminosäuren 242 bis 261 der Sequenz definiert ist, die in Figur 14A, 14C oder 15A gezeigt ist, in die ein Einsatz eingeführt wurde, wobei der Einsatz aus einer variablen Domänensequenz eines Fc-Moleküls besteht.

## Revendications

1. Bibliothèque de protéines combinatoire comprenant une pluralité d'espèces protéiques, chaque espèce protéique comprenant une chaîne A d'une protéine qui est une toxine Shiga-like I, modifiée par mutation de l'acide aminé Cys242 et/ou de l'acide aminé Cys 261 de la séquence représentée dans la Figure 14A, la Figure 14C, ou la Figure 15A, la chaîne A modifiée comprenant en outre une boucle sensible aux protéases telle que définie en référence aux acides aminés 242 à 261 de la séquence représentée dans la Figure 14A, la Figure 14C, ou la Figure 15A, dans laquelle un insert a été introduit, l'insert comprenant un polypeptide de séquence d'acides aminés variable ayant une longueur de 3 à 200 résidus acides aminés.

2. Bibliothèque de protéines combinatoire selon la revendication 1, dans laquelle la chaîne A modifiée comprend un résidu cystéine et comprend en outre une étiquette ou une sonde liée au résidu cystéine.

3. Bibliothèque de protéines combinatoire selon la revendication 1 ou 2, dans laquelle la mutation dans au moins une cystéine est une mutation d'une cystéine à une alanine.

4. Bibliothèque de protéines combinatoire selon l'une quelconque des revendications précédentes, dans laquelle l'espèce protéique est formée par introduction de l'insert entre les acides aminés 245 et 246, tels que définis en référence à la séquence représentée dans la Figure 14A, la Figure 14C, ou la Figure 15A.

5. Bibliothèque de protéines combinatoire selon l'une quelconque des revendications précédentes, dans laquelle l'insert a une longueur de 7 acides aminés.

6. Protéine mutante comprenant une chaîne A d'une protéine qui est une toxine Shiga-like I, modifiée par mutation de l'acide aminé Cys242 et/ou de l'acide aminé Cys 261 de la séquence représentée dans la Figure 14A, la Figure 14C, ou la Figure 15A, la chaîne A modifiée comprenant en outre une boucle sensible aux protéases telle que définie en référence aux acides aminés 242 à 261 de la séquence représentée dans la Figure 14A, la Figure 14C, ou la Figure 15A, dans laquelle un insert a été introduit, l'insert comprenant un polypeptide de séquence d'acides aminés variable ayant une longueur de 3 à 200 résidus acides aminés.

7. Protéine mutante selon la revendication 6, dans laquelle la mutation dans au moins une cystéine est une mutation d'une cystéine à une alanine.

8. Protéine mutante selon la revendication 6, dans laquelle l'insert est introduit entre les acides aminés 245 et 246, tels que définis en référence à la séquence représentée dans la Figure 14A, la Figure 14C, ou la Figure 15A.

9. Protéine mutante selon la revendication 8, dans laquelle l'insert comprend la séquence IYSNKLM.

10. Protéine mutante selon la revendication 8, dans laquelle l'insert comprend la séquence AAFADLI.

11. Procédé d'isolation d'une toxine spécifique pour une cible/un récepteur, comprenant les étapes qui consistent à :
(a) exposer la cible/le récepteur à une bibliothèque de protéines combinatoire selon l'une quelconque des revendications 1 à 5 ; et
(b) isoler au moins une espèce protéique à partir de la bibliothèque de protéines combinatoire, capturée par liaison à la cible/au récepteur.

12. Procédé selon la revendication 11, comprenant en outre l'étape de criblage de la protéine isolée contre des cellules exprimant la cible/le récepteur, pour confirmer sa toxicité pour des cellules exprimant la cible/le récepteur.

13. Procédé selon la revendication 11, dans lequel la cible/le récepteur est une cible/un récepteur purifié(e) et est immobilisé(e) sur un support solide.

14. Procédé selon la revendication 11, dans lequel la cible/le récepteur est sur la surface de cellules.

15. Procédé selon la revendication 14, dans lequel les cellules sont immobilisées sur un support solide.

16. Procédé selon l'une quelconque des revendications 12 à 15, dans lequel la toxine sert de reporter, et la mort des cellules indique une liaison au récepteur.

17. Procédé selon l'une quelconque des revendications 12 à 15, dans lequel les cellules sont des cellules cancéreuses.

18. Bibliothèque de protéines combinatoire selon la revendication 2, dans laquelle la chaîne A modifiée est une chaîne A modifiée de toxine Shiga-like I, et dans laquelle les étiquettes ou les sondes sont sélectionnées dans un groupe constitué d'étiquettes ou de sondes spectroscopiques, radioactives, et biochimiques.

19. Bibliothèque de protéines combinatoire comprenant une pluralité d'espèces protéiques, chaque espèce protéique comprenant une chaîne A d'une protéine qui est une toxine Shiga-like I, modifiée par mutation de l'acide aminé Cys242 et/ou de l'acide aminé Cys 261 de la séquence représentée dans la Figure 14A, la Figure 14C, ou la Figure 15A, la chaîne A modifiée comprenant en outre une boucle sensible aux protéases telle que définie en référence aux acides aminés 242 à 261 de la séquence représentée dans la Figure 14A, la Figure 14C, ou la Figure 15A, dans laquelle un insert a été introduit, l'insert consistant en une séquence de domaine variable d'une molécule Fc.
